# EUROPEAN PATENT APPLICATION

(11) **EP 2 810 955 A1**
(43) Date of publication of application: **10.12.2014**
(21) Application number: 13305762.0
(22) Date of filing: 07.06.2013
(51) Int. Cl.: C07K 16/40

(54) **Methods for inhibiting atherosclerosis by administering an inhibitor of PCSK9**

(71) Applicant: SANOFI, 75008 Paris (FR)
(72) Inventor: The designation of the inventor has not yet been filed
(74) Representative: Then, Johann

(57) **Abstract**

The present invention provides methods and compositions for inhibiting atherosclerotic plaque formation in a subject. In certain embodiments, the methods of the present invention comprise selecting a subject who has, or is at risk of developing, atherosclerosis, and administering to the subject a pharmaceutical composition comprising a proprotein convertase subtilisin/kexin type 9 (PCSK9) inhibitor. In certain embodiments, the PCSK9 inhibitor is an anti-PCSK9 antibody, or antigen binding fragment thereof.

## Description

### FIELD OF THE INVENTION

The present invention relates to the field of therapeutic treatments for atherosclerosis. More specifically, the invention relates to the administration ofproprotein convertase subtilisin/kexin type 9 (PCSK9) inhibitors to inhibit atherosclerotic plaque formation in a subject.

### BACKGROUND

Atherosclerosis represents the major cause of death and cardiovascular morbidity in the western world. Risk factors for atherosclerosis include high low density lipoprotein (LDL) cholesterol levels, low high density lipoprotein (HDL) cholesterol levels, hypertension, diabetes mellitus, family history, male gender, cigarette smoke, and high serum cholesterol.

Proprotein convertase subtilisin/kexin type 9 (PCSK9) is a proprotein convertase belonging to the proteinase K subfamily of the secretory subtilase family. Evidence suggest that PCSK9 increases plasma LDL cholesterol by promoting degradation of the LDL receptor, which mediates LDL endocytosis in the liver, the major route of LDL clearance from circulation.

The use of PCSK9 inhibitors (anti-PCSK9 antibodies) to reduce serum total cholesterol, LDL cholesterol and serum triglycerides has been described in US Patent Appl. Publ. No. 2010/0166768. Nonetheless, PCSK9 inhibitors have not been reported to reduce or inhibit progression of atherosclerotic plaque formation in a subject. There remains a need in the art for therapeutic methods of inhibiting atherosclerotic plaque formation.

### BRIEF SUMMARY OF THE INVENTION

The present invention addresses the need in the art for therapeutic methods by providing methods and compositions for inhibiting atherosclerotic plaque formation in a subject. In certain aspects, the methods of the present invention generally comprise selecting a subject who has, or is at risk of developing, atherosclerosis, and administering to the subject a pharmaceutical composition comprising a proprotein convertase subtilisin/kexin type 9 (PCSK9) inhibitor (e.g., an anti-PCSK9 antibody or antigen binding fragment thereof). In other aspects, the invention provides a pharmaceutical composition comprising a PCSK9 inhibitor for use in the inhibition of atherosclerotic plaque formation in a subject. In yet other aspects, the invention provides a method of inhibiting atherosclerotic plaque formation in a subject, the method comprising administering to the subject a pharmaceutical composition comprising a PCSK9 inhibitor.

In certain embodiments, the subject is nonhyperlipidemic. In other embodiments, the subject is apparently healthy.

In another aspect, the invention provides a method of treating or inhibiting progression of atherosclerosis in a subject, the method comprising: (a) selecting a subject who has suffered a stroke or myocardial infarction; and (b) administering to the subject a pharmaceutical composition comprising a PCSK9 inhibitor, thereby treating or inhibiting progression of atherosclerosis. In one embodiment, the subject is nonhyperlipidemic.

In another aspect, the invention provides a method of treating or inhibiting progression of atherosclerosis in a nonhyperlipidemic subject, the method comprising (a) selecting a subject who has, or is known to be at risk of developing, atherosclerosis, wherein the subject is nonhyperlipidemic; and (b) administering to the subject a pharmaceutical composition comprising a PCSK9 inhibitor, thereby treating or inhibiting progression of atherosclerosis. In one embodiment, the subject is apparently healthy. In another embodiment, the subject is nonhypercholesterolemic. In another embodiment, the subject is nonhypertriglyceridemic.

In certain embodiments, the subject has a disease or disorder selected from the group consisting of type I diabetes mellitus, type II diabetes mellitus, Kawasaki disease, chronic inflammatory disease, and hypertension. In another embodiment, the subject has elevated levels of an inflammatory marker. In a further embodiment, the inflammatory marker is C-reactive protein. In another further embodiment, the inflammatory marker is an inflammatory cytokine, In certain exemplary embodiments, the PCSK9 inhibitor is an antibody, or antigen binding fragment thereof, that specifically binds to PCSK9.

In other exemplary embodiments, the antibody, or antigen binding fragment thereof, exhibits one or more or the following properties when administered by weekly subcutaneous injection to a APOE*3Leiden.CETP mouse:
a. reduces total cholesterol levels relative to untreated controls by about 37% when administered at 3 mg/kg;
b. reduces total cholesterol levels relative to untreated controls by about 46% when administered at 10 mg/kg;
c. reduces total cholesterol levels relative to untreated controls by about 48% when administered at 3 mg/kg in combination with 3.6 mg/kg/day atorvastin;
d. reduces total cholesterol levels relative to untreated controls by about 58% when administered at 10 mg/kg in combination with 3.6 mg/kg/day atorvastin;
e. reduces total cholesterol levels by about 36% when administered at 3 mg/kg in combination with 3.6 mg/kg/day atorvastin, relative to treatment with 3.6 mg/kg/day atorvastin alone;
f. reduces total cholesterol levels by about 48% when administered at 10 mg/kg in combination with 3.6 mg/kg/day atorvastin, relative to treatment with 3.6 mg/kg/day atorvastin alone;
g. reduces triglyceride levels relative to untreated controls by about 34% when administered at 3 mg/kg;
h. reduces triglyceride levels relative to untreated controls by about 51% when administered at 10 mg/kg;
i. reduces triglyceride levels by about 51% when administered at 3 mg/kg in combination with 3.6 mg/kg/day atorvastin, relative to treatment with 3.6 mg/kg/day atorvastin;
j. reduces triglyceride levels by about 57% when administered at 10 mg/kg in combination with 3.6 mg/kg/day atorvastin, relative to treatment with 3.6 mg/kg/day atorvastin alone;
k. increases hepatic LDLR expression relative to untreated controls by about 95% when administered at 3 mg/kg;
1. increases hepatic LDLR expression relative to untreated controls by about 133% when administered at 10 mg/kg;
m. increases hepatic LDLR expression by about 106% when administered at 3 mg/kg in combination with 3.6 mg/kg/day atorvastin, relative to treatment with 3.6 mg/kg/day atorvastin alone;
n. increases hepatic LDLR expression by about 165% when administered at 10 mg/kg in combination with 3.6 mg/kg/day atorvastin, relative to treatment with 3.6 mg/kg/day atorvastin alone;
o. decreases atherosclerotic lesion size relative to untreated controls by about 70% when administered at 3 mg/kg;
p. decreases atherosclerotic lesion size relative to untreated controls by about 87% when administered at 10 mg/kg;
q. decreases atherosclerotic lesion size relative to untreated controls by about 88% when administered at 3 mg/kg in combination with 3.6 mg/kg/day atorvastin;
r. decreases atherosclerotic lesion size relative to untreated controls by about 98% when administered at 10 mg/kg in combination with 3.6 mg/kg/day atorvastin;
s. decreases atherosclerotic lesion size relative by about 82% when administered at 3 mg/kg in combination with 3.6 mg/kg/day atorvastin, relative to treatment with 3.6 mg/kg/day atorvastin alone;
t. decreases atherosclerotic lesion size relative by about 97% when administered at 10 mg/kg in combination with 3.6 mg/kg/day atorvastin, relative to treatment with 3.6 mg/kg/day atorvastin alone;
u. reduces triglyceride levels by about 72% when administered at 3 mg/kg, relative to treatment with 3.6 mg/kg/day atorvastatin alone;
v. reduces triglyceride levels by about 79% when administered at 10 mg/kg, relative to treatment with 3.6 mg/kg/day atorvastatin alone;
w. reduces total cholesterol levels by about 22% when administered at 3 mg/kg, relative to treatment with 3.6 mg/kg/day atorvastatin alone;
x. reduces total cholesterol levels by about 34% when administered at 10 mg/kg, relative to treatment with 3.6 mg/kg/day atorvastatin alone;
y. increases the percentage of undiseased aortic segments by about 236% when administered at 3 mg/kg, relative to untreated control;
z. increases the percentage of undiseased aortic segments by about 549% when administered at 10 mg/kg, relative to untreated control;
aa. increases the percentage of undiseased aortic segments by about 607% when administered at 3 mg/kg in combination with 3.6 mg/kg/day atorvastatin, relative to control; and
bb. increases the percentage of undiseased aortic segments by about 1118% when administered at 3 mg/kg in combination with 3.6 mg/kg/day atorvastatin, relative to control.

In certain exemplary embodiments, the antibody comprises: a heavy chain variable domain comprising the CDR amino acid sequences set forth in SEQ ID NOs: 76, 78, and 80; and a light chain variable domain comprising the CDR amino acid sequences set forth in SEQ ID NOs: 84, 86 and 88. In another exemplary embodiment, the antibody comprises: a heavy chain variable domain comprising the CDR amino acid sequences set forth in SEQ ID NOs: 220, 222, and 224; and a light chain variable domain comprising the CDR amino acid sequences set forth in SEQ ID NOs: 802, 230 and 232. In another exemplary embodiment, the antibody comprises the heavy chain variable domain and the light chain variable domain respectively comprise the amino acid sequences set forth in SEQ ID NOs 90 and 92. In another exemplary embodiment, the antibody comprises the heavy chain variable domain and the light chain variable domain respectively comprise the amino acid sequences set forth in SEQ ID NOs 218 and 216.

In one embodiment, the antibody or antigen-binding fragment thereof binds to the same epitope on PCSK9 as an antibody comprising the heavy and light variable domain amino acid sequences forth in SEQ ID NOs: 90 and 92; or 218 and 216, respectively. In another embodiment, the antibody or antigen-binding fragment thereof competes for binding to PCSK9 with an antibody comprising the heavy and light chain variable domain amino acid sequences forth in SEQ ID NOs: 90 and 92; or 218 and 216, respectively.

In another embodiment, the PCSK9 inhibitor reduces atherosclerotic plaque formation in the subject by at least 10%, 20%, 30%, 40%, 50%, 60%, 70%, 80%, or 90%.

In certain embodiments, the PCSK9 inhibitor is administered in combination with a statin. In a further embodiment, the therapeutic statin is selected from the group consisting of cerivastatin, atorvastatin, simvastatin, pitavastatin, rosuvastatin, fluvastatin, lovastatin and pravastatin. In an exemplary embodiment, the statin is atorvastatin.

### BRIEF DESCRIPTION OF THE DRAWINGS

Figure 1 depicts the timeline of a mouse study examining the effects of different drug regimens on APOE*Leiden.CETP mice on a Western-Type Diet (WTD).
Figure 2 depicts a graph of the average total cholesterol level (mM) in different treatment cohorts over the course of an 18 week mouse study.
Figure 3 depicts a graph of the average triglyceride level (mM) in different treatment cohorts over the course of an 18 week mouse study.
Figure 4 depicts a graph of the total cholesterol exposure of animals in different treatment cohorts at 18 weeks.
Figure 5 depicts a graph of the average LDLR protein expression and the averaged ratio of LDLR protein to tubulin protein expression in different treatment populations relative to control.
Figure 6 depicts a graph of the average atherosclerotic lesion area per cross section (*1000 µm) in different treatment groups.
Figure 7 depicts a graph of the average atherosclerotic lesion severity observed in different treatment groups (as a percentage of all lesions) broken down into mild lesions (type I-III) and severe lesions (types IV and V).
Figure 8 depicts a graph of the average number of atherosclerotic lesions observed in different treatment groups broken down by lesion type (Type I-V and Type I-V and valve).
Figure 9 depicts a graph of the average atherosclerotic lesion area per cross section (*1000 µm) observed in different treatment groups broken down by lesion type (Type I-III, Type IV-V, and Type I-V).
Figure 10 depicts atherosclerotic lesions observed in different treatment cohorts after 18 weeks.
Figure 11 depicts a graph of the percentage of undiseased aortic segments observed in each treatment cohort after 18 weeks.

### DETAILED DESCRIPTION

Before the present invention is described, it is to be understood that this invention is not limited to particular methods and experimental conditions described, as such methods and conditions may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. As used herein, the term "about," when used in reference to a particular recited numerical value, means that the value may vary from the recited value by no more than 1%. For example, as used herein, the expression "about 100" includes 99 and 101 and all values in between (e.g., 99.1, 99.2, 99.3, 99.4, etc.).

Although any methods and materials similar or equivalent to those described herein can be used in the practice of the present invention, exemplary methods and materials are now described. All publications mentioned herein, and the sequence listing filed concurrently herewith, are incorporated herein by reference in their entirety.

### I. Patient Populations

In certain aspects, the methods of the present invention comprise selecting subjects that have, or are at risk of developing, atherosclerosis, and administering to these subjects a pharmaceutical composition comprising a PCSK9 inhibitor.

Risk factors for atherosclerosis are well known in the art and include, without limitation, high low density lipoprotein (LDL) cholesterol levels, low high density lipoprotein (HDL) cholesterol levels, hypertension, diabetes mellitus, family history, male gender, cigarette smoking, and high serum cholesterol. Methods of assessing these risk factors for a given subject are also well known in the art.

In certain embodiments, the selected subject is nonhyperlipidemic. A "nonhyperlipidemic" is a subject that is a nonhypercholesterolemic and/or a nonhypertriglyceridemic subject. A "nonhypercholesterolemic" subject is one that does not fit the current criteria established for a hypercholesterolemic subject. A "nonhypertriglyceridemic" subject is one that does not fit the current criteria established for a hypertriglyceridemic subject (See, e.g., Harrison's Principles of Experimental Medicine, 13th Edition, McGraw-Hill, Inc., N.Y). A hypercholesterolemic subject has an LDL level of >160 mg/dL, or >130 mg/dL and at least two risk factors selected from the group consisting of male gender, family history of premature coronary heart disease, cigarette smoking (more than 10 per day), hypertension, low HDL (<35 mg/dL), diabetes mellitus, hyperinsulinemia, abdominal obesity, high lipoprotein (a), and personal history of cerebrovascular disease or occlusive peripheral vascular disease. A hypertriglyceridemic subject has a triglyceride (TG) level of >250 mg/dL. Thus, a nonhyperlipidemic subject is defined as one whose cholesterol and triglyceride levels are below the limits set as described above for both the hypercholesterolemic and hypertriglyceridemic subjects. In certain embodiments the selected subject is neither nonhyperlipidemic nor receiving treatment for hyperlipidemia.

In certain embodiments, the selected subject is apparently healthy. "Apparently healthy", as used herein, means individuals who have not previously had an acute, adverse cardiovascular event such as a myocardial infarction (i.e., individuals who are not at an elevated risk of a second adverse cardiovascular event due to a primary adverse cardiovascular event). Apparently healthy individuals also do not otherwise exhibit symptoms of disease.

In certain embodiments, the selected subject has previously suffered an acute adverse cardiovascular event such as a myocardial infarction, stroke, angina pectoris and/or peripheral arteriovascular disease. In one embodiment, the selected subject has previously suffered an acute adverse cardiovascular event such as a myocardial infarction, stroke, angina pectoris and/or peripheral arteriovascular disease, but is nonhyperlipidemic. In one embodiment, the selected subject has previously suffered an acute adverse cardiovascular event such as a myocardial infarction, stroke, angina pectoris and/or peripheral arteriovascular disease, but is neither nonhyperlipidemic nor receiving treatment for hyperlipidemia.

In certain embodiments, the selected subject has a disease or disorder selected from the group consisting of type I diabetes mellitus, type II diabetes mellitus, Kawasaki disease, chronic inflammatory disease, and hypertension. In one embodiment, the selected subject has a disease or disorder selected from the group consisting of type I diabetes mellitus, type II diabetes mellitus, Kawasaki disease, chronic inflammatory disease, and hypertension, but is nonhyperlipidemic. In one embodiment, the selected subject has a disease or disorder selected from the group consisting of type I diabetes mellitus, type II diabetes mellitus, Kawasaki disease, chronic inflammatory disease, and hypertension, but is neither nonhyperlipidemic nor receiving treatment for hyperlipidemia.

In certain embodiments, the selected subject has elevated levels of an inflammatory marker. In one embodiment, the selected subject has elevated levels of an inflammatory marker, but is nonhyperlipidemic. Any marker of systemic inflammation can be utilized for the purposes of the present invention. Suitable inflammatory markers include, without limitation, C-reactive protein (see e.g., US Patent Number 7,964,614, which is incorporated by reference herein in its entirety), cytokines (e.g., Il-6, IL-8, and/or IL-17), and cellular adhesion molecules (e.g., ICAM-1, ICAM-3, BL-CAM, LFA-2, VCAM-1, NCAM, and PECAM).

The level of an inflammatory marker can be obtained by any art-recognized assay. Typically, the level is determined by measuring the level of the marker in a body fluid, for example, blood, lymph, saliva, urine and the like. The level can be determined by ELISA, or immunoassays or other conventional techniques for determining the presence of the marker. To determine if levels of the inflammatory marker are elevated, the level of the marker measured in a subject can be compared to a suitable control (e.g., a predetermined value and/or a value obtained from a matched healthy subject).

### II. PCSK9 Inhibitors

In certain aspects, the methods of the present invention comprise administering to a subject a therapeutic composition comprising a PCSK9 inhibitor.

As used herein, a "PCSK9 inhibitor" is any agent which binds to or interacts with human PCSK9 and inhibits the normal biological function of PCSK9 *in vitro* or *in vivo.* Non-limiting examples of categories of PCSK9 inhibitors include small molecule PCSK9 antagonists, antagonistic nucleic acid molecules (e.g., RNAi molecules) peptide-based PCSK9 antagonists (e.g., "peptibody" molecules), and antibodies or antigen-binding fragments of antibodies that specifically bind human PCSK9. Exemplary, non-limiting, PCSK9 inhibitors are set forth herein and, for example, in US20130115223A1, US20130071405 A1, US20130064834A1, US20130064825A1, US20120122954A1, US20120015435A1, US20110313024A1, US 20110015252A1, US20110230542A1, US20110009628A1, and US Serial Number 61/682,349, which are each incorporated herein by reference in their entireties.

As used herein, the term "proprotein convertase subtilisin/kexin type 9" or "PCSK9" refers to PCSK9 having the nucleic acid sequence shown in SEQ ID NO:754 and the amino acid sequence of SEQ ID NO:755, or a biologically active fragment thereof.

In certain embodiments, administration of the PCSK9 inhibitor reduces atherosclerotic plaque formation in the subject (e.g., a human subject) by at least 10% (e.g., 10% 20%, 30%, 40%, 50%, 60%, 70%, 80%, 90%, or 100%) relative to atherosclerotic plaque formation in an untreated subject.

In certain embodiments, the PCSK9 inhibitor is an antibody or antigen-binding fragment thereof that specifically bind to PCSK9. As used herein, the term "antibody" refers to immunoglobulin molecules comprising four polypeptide chains, two heavy (H) chains and two light (L) chains inter-connected by disulfide bonds, as well as multimers thereof (e.g., IgM). Each heavy chain comprises a heavy chain variable region (abbreviated herein as HCVR or V_{H}) and a heavy chain constant region. The heavy chain constant region comprises three domains, C_{H}1, C_{H}2 and C_{H}3. Each light chain comprises a light chain variable region (abbreviated herein as LCVR or V_{L}) and a light chain constant region. The light chain constant region comprises one domain (C C_{L}1). The V_{H} and V_{L} regions can be further subdivided into regions of hypervariability, termed complementarity determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. In different embodiments of the invention, the FRs of the anti-PCSK9 antibody (or antigen-binding portion thereof) may be identical to the human germline sequences, or may be naturally or artificially modified. An amino acid consensus sequence may be defined based on a side-by-side analysis of two or more CDRs.

The term "antibody," as used herein, also includes antigen-binding fragments of full antibody molecules. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, e.g., commercial sources, DNA libraries (including, e.g., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (e.g., an isolated complementarity determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (e.g. monovalent nanobodies, bivalent nanobodies, etc.), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR which is adjacent to or in frame with one or more framework sequences. In antigen-binding fragments having a V_{H} domain associated with a V_{L} domain, the V_{H} and V_{L} domains may be situated relative to one another in any suitable arrangement. For example, the variable region may be dimeric and contain V_{H}-V_{H}, V_{H}-V_{L} or V_{L}-V_{L} dimers. Alternatively, the antigen-binding fragment of an antibody may contain a monomeric V_{H} or V_{L} domain.

In certain embodiments, an antigen-binding fragment of an antibody may contain at least one variable domain covalently linked to at least one constant domain. Non-limiting, exemplary configurations of variable and constant domains that may be found within an antigen-binding fragment of an antibody of the present invention include: (i) V_{H}-C_{H}I; (ii) V_{H} - C_{H}2; (iii) V_{H} - C_{H}3; (iv) V_{H}-C_{H}1-C_{H}2; (v) V_{H}-C_{H}1-C_{H}2-C_{H}3; (vi) V_{H}-C_{H}2-C_{H}3; (vii) V_{H}-C_{L}; (viii) V_{L}-C_{H}1; (ix) V_{L},-C_{H}2; (x) V_{L}-C_{H}3, (xi) V_{L}-C_{H}I-C_{H}2; (xii) V_{L}-C_{H}I-C_{H}2-C_{H}3; (xiii) V_{L}-C_{H}2-C_{H}3; and (xiv) V_{L}-C_{L}. In any configuration of variable and constant domains, including any of the exemplary configurations listed above, the variable and constant domains may be either directly linked to one another or may be linked by a full or partial hinge or linker region. A hinge region may consist of at least 2 (e.g., 5, 10, 15, 20, 40, 60 or more) amino acids which result in a flexible or semi-flexible linkage between adjacent variable and/or constant domains in a single polypeptide molecule.

Moreover, an antigen-binding fragment of an antibody of the present invention may comprise a homo-dimer or hetero-dimer (or other multimer) of any of the variable and constant domain configurations listed above in non-covalent association with one another and/or with one or more monomeric V_{H} or V_{L} domain (e.g., by disulfide bond(s)).

As with full antibody molecules, antigen-binding fragments may be monospecific or multispecific (e.g., bispecific). A multispecific antigen-binding fragment of an antibody will typically comprise at least two different variable domains, wherein each variable domain is capable of specifically binding to a separate antigen or to a different epitope on the same antigen. Any multispecific antibody format, including the exemplary bispecific antibody formats disclosed herein, may be adapted for use in the context of an antigen-binding fragment of an antibody of the present invention using routine techniques available in the art.

The constant region of an antibody is important in the ability of an antibody to fix complement and mediate cell-dependent cytotoxicity. Thus, the isotype of an antibody may be selected on the basis of whether it is desirable for the antibody to mediate cytotoxicity.

The term "human antibody", as used herein, is intended to include antibodies having variable and constant regions derived from human germline immunoglabulin sequences. The human antibodies of the invention may nonetheless include amino acid residues not encoded by human germline immunoglobulin sequences (e.g., mutations introduced by random or site-specific mutagenesis *in vitro* or by somatic mutation *in vivo),* for example in the CDRs and in particular CDR3. However, the term "human antibody", as used herein, is not intended to include antibodies in which CDR sequences derived from the germline of another mammalian species, such as a mouse, have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see e.g., Taylor et al. (1992) Nucl. Acids Res. 20:6287-6295) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and V_{L} regions of the recombinant antibodies are sequences that, while derived from and related to human germline VH and V_{L} sequences, may not naturally exist within the human antibody germline repertoire *in vivo.*

Human antibodies can exist in two forms that are associated with hinge heterogeneity. In one form, an immunoglobulin molecule comprises a stable four chain construct of approximately 150-160 kDa in which the dimers are held together by an interchain heavy chain disulfide bond. In a second form, the dimers are not linked via interchain disulfide bonds and a molecule of about 75-80 kDa is formed composed of a covalently coupled light and heavy chain (half-antibody). These forms have been extremely difficult to separate, even after affinity purification.

The frequency of appearance of the second form in various intact IgG isotypes is due to, but not limited to, structural differences associated with the hinge region isotype of the antibody. A single amino acid substitution in the hinge region of the human IgG4 hinge can significantly reduce the appearance of the second form (see e.g., Angal et al. (1993) Molecular Immunology 30:105) to levels typically observed using a human IgG1 hinge. The instant invention encompasses antibodies having one or more mutations in the hinge, CH2 or CH3 region which may be desirable, for example, in production, to improve the yield of the desired antibody form.

An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody" for purposes of the present invention. An isolated antibody also includes an antibody *in situ* within a recombinant cell. Isolated antibodies are antibodies that have been subjected to at least one purification or isolation step. According to certain embodiments, an isolated antibody may be substantially free of other cellular material and/or chemicals.

The term "specifically binds," or the like, means that an antibody or antigen-binding fragment thereof forms a complex with an antigen that is relatively stable under physiologic conditions. Methods for determining whether an antibody specifically binds to an antigen are well known in the art and include, for example, equilibrium dialysis, surface plasmon resonance, and the like. For example, an antibody that "specifically binds" PCSK9, as used in the context of the present invention, includes antibodies that bind PCSK9 or portion thereof with a KD of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM or less than about 0.5 nM, as measured in a surface plasmon resonance assay. An isolated antibody that specifically binds human PCSK9 can, however, have cross-reactivity to other antigens, such as PCSK9 molecules from other (non-human) species.

Anti-PCSK9 antibodies useful in the methods and compositions of the present invention may comprise one or more amino acid substitutions, insertions and/or deletions in the framework and/or CDR regions of the heavy and light chain variable domains as compared to the corresponding germline sequences from which the antibodies were derived. Such mutations can be readily ascertained by comparing the amino acid sequences disclosed herein to germline sequences available from, for example, public antibody sequence databases. The present invention includes methods involving the use of antibodies, and antigen-binding fragments thereof, which are derived from any of the amino acid sequences disclosed herein, wherein one or more amino acids within one or more framework and/or CDR regions are mutated to the corresponding residue(s) of the germline sequence from which the antibody was derived, or to the corresponding residue(s) of another human germline sequence, or to a conservative amino acid substitution of the corresponding germline residue(s) (such sequence changes are referred to herein collectively as "germline mutations"). A person of ordinary skill in the art, starting with the heavy and light chain variable region sequences disclosed herein, can easily produce numerous antibodies and antigen-binding fragments which comprise one or more individual germline mutations or combinations thereof. In certain embodiments, all of the framework and/or CDR residues within the VH and/or V_{L} domains are mutated back to the residues found in the original germline sequence from which the antibody was derived. In other embodiments, only certain residues are mutated back to the original germline sequence, *e.g*.,only the mutated residues found within the first 8 amino acids of FR1 or within the last 8 amino acids of FR4, or only the mutated residues found within CDR1, CDR2 or CDR3. In other embodiments, one or more of the framework and/or CDR residue(s) are mutated to the corresponding resiclue(s) of a different germline sequence *(i.e.,* a germline sequence that is different from the germline sequence from which the antibody was originally derived). Furthermore, the antibodies of the present invention may contain any combination of two or more germline mutations within the framework and/or CDR regions, e.g., wherein certain individual residues are mutated to the corresponding residue of a particular germline sequence while certain other residues that differ from the original germline sequence are maintained or are mutated to the corresponding residue of a different germline sequence. Once obtained, antibodies and antigen-binding fragments that contain one or more germline mutations can be easily tested for one or more desired property such as, improved binding specificity, increased binding affinity, improved or enhanced antagonistic or agonistic biological properties (as the case may be), reduced immunogenicity, etc. The use of antibodies and antigen-binding fragments obtained in this general manner are encompassed within the present invention.

The present invention also includes methods involving the use of anti-PCSK9 antibodies comprising variants of any of the HCVR, LCVR, and/or CDR amino acid sequences disclosed herein having one or more conservative substitutions. For example, the present invention includes the use of anti-PCSK9 antibodies having HCVR, LCVR, and/or CDR amino acid sequences with, e.g., 10 or fewer, 8 or fewer, 6 or fewer, 4 or fewer, etc. conservative amino acid substitutions relative to any of the HCVR, LCVR, and/or CDR amino acid sequences disclosed herein.

The term "surface plasmon resonance", as used herein, refers to an optical phenomenon that allows for the analysis of real-time interactions by detection of alterations in protein concentrations within a biosensor matrix, for example using the BIAcore™ system (Biacore Life Sciences division of GE Healthcare, Piscataway, NJ).

The term "K_{D}", as used herein, is intended to refer to the equilibrium dissociation constant of a particular antibody-antigen interaction.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

### III. Preparation of Human Antibodies

Methods for generating human antibodies in transgenic mice are known in the art. Any such known methods can be used in the context of the present invention to make human antibodies that specifically bind to human PCSK9.

Using VELOCIMMUNE™ technology (see, for example, US 6,596,541, Regeneron Pharmaceuticals) or any other known method for generating monoclonal antibodies, high affinity chimeric antibodies to PCSK9 are initially isolated having a human variable region and a mouse constant region. The VELOCIMMUNE® technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody.

Generally, a VELOCIMMUNE® mouse is challenged with the antigen of interest, and lymphatic cells (such as B-cells) are recovered from the mice that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. DNA encoding the variable regions of the heavy chainand light chain may be isolated and linked to desirable isotypic constant regions of the heavy chain and light chain. Such an antibody protein may be produced in a cell, such as a CHO cell. Alternatively, DNA encoding the antigen-specific chimeric antibodies or the variable domains of the light and heavy chains may be isolated directly from antigen-specific lymphocytes.

Initially, high affinity chimeric antibodies are isolated having a human variable region and a mouse constant region. The antibodies are characterized and selected for desirable characteristics, including affinity, selectivity, epitope, etc, using standard procedures known to those skilled in the art. The mouse constant regions are replaced with a desired human constant region to generate the fully human antibody of the invention, for example wild-type or modified IgG1 or IgG4. While the constant region selected may vary according to specific use, high affinity antigen-binding and target specificity characteristics reside in the variable region.

In general, the antibodies that can be used in the methods of the present invention possess high affinities, as described above, when measured by binding to antigen either immobilized on solid phase or in solution phase. The mouse constant regions are replaced with desired human constant regions to generate the fully human antibodies of the invention. While the constant region selected may vary according to specific use, high affinity antigen-binding and target specificity characteristics reside in the variable region.

Specific examples of human antibodies or antigen-binding fragments of antibodies that specifically bind PCSK9 which can be used in the context of the methods of the present invention include any antibody or antigen-binding fragment which comprises the three heavy chain CDRs (HCDR1, HCDR2 and HCDR3) contained within a heavy chain variable region (HCVR) having an amino acid sequence selected from the group consisting of SEQ ID NOs: 2, 18, 22, 26, 42, 46, 50, 66, 70, 74, 90, 94, 98, 114, 118, 122, 138, 142, 146, 162, 166, 170, 186, 190, 194, 210, 214, 218,234,238,242,258,262,266,282,286,290,306,310,314,330,334,338,354,358,362, 378, 382, 386, 402, 406, 410, 426, 430, 434, 450, 454, 458, 474, 478, 482, 498, 502, 506, 522, 526, 530, 546, 550, 554, 570, 574, 578, 594, 598, 602, 618, 622, 626, 642, 646, 650, 666, 670, 674, 690, 694, 698, 714, 718, 722, 738 and 742, or a substantially similar sequence thereof having at least 90%, at least 95%, at least 98% or at least 99% sequence identity. The antibody or antigen-binding fragment may comprise the three light chain CDRs (LCVR1, LCVR2, LCVR3) contained within a light chain variable region (LCVR) having an amino acid sequence selected from the group consisting of SEQ ID NOs: 10, 20, 24, 34, 44, 48, 58, 68, 72, 82, 92, 96, 106, 116, 120, 130, 140, 144, 154, 164, 168, 178, 188, 192, 202, 212, 216, 226, 236, 240, 250, 260, 264, 274, 284, 288, 298, 308, 312, 322, 332, 336, 346, 356, 360, 370, 380, 384, 394, 404, 408, 418, 428, 432, 442, 452, 456, 466, 476, 480, 490, 500, 504, 514, 524, 528, 538, 548, 552, 562, 572, 576, 586, 596, 600, 610, 620, 624, 634, 644, 648, 658, 668, 672, 682, 692, 696, 706, 716, 720, 730, 740 and 744, or a substantially similar sequence thereof having at least 90%, at least 95%, at least 98% or at least 99% sequence identity.

In certain embodiments of the present invention, the antibody or antigen-binding fragment thereof comprises the six CDRs (HCDR1, HCDR2, HCDR3, LCDR1, LCDR2 and LCDR3) from the heavy and light chain variable region amino acid sequence pairs (HCVR/LCVR) selected from the group consisting of SEQ ID NOs: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, 262/264, 266/274, 282/284, 286/288, 290/298, 306/308, 310/312, 314/322, 330/332, 334/336, 338/346, 354/356, 358/360, 362/370, 378/380, 382/384, 386/394, 402/404, 406/408, 410/418, 426/428, 430/432, 434/442, 450/452, 454/456, 458/466, 474/476, 478/480, 482/490, 498/500, 502/504, 506/514, 522/524, 526/528, 530/538, 546/548, 550/552, 554/562, 570/572, 574/576, 578/586, 594/596, 598/600, 602/610, 618/620, 622/624, 626/634, 642/644, 646/648, 650/658, 666/668, 670/672, 674/682, 690/692, 694/696, 698/706, 714/716, 718/720, 722/730, 738/740 and 742/744.

In certain embodiments of the present invention, the anti-PCSK9 antibody, or antigen-binding fragment thereof, that can be used in the methods of the present invention has HCDR1/HCDR2/HCDR3/LCDR1/LCDR2/LCDR3 amino acid sequences selected from SEQ ID NOs: 76/78/80/84/86/88 (mAb316P) and 220/222/224/228/230/232 (mAb300N) (*See* US Patent App. Publ No. 2010/0166768) and 220/222/224/802/230/232.

In certain embodiments of the present invention, the antibody or antigen-binding fragment thereof comprises HCVR/LCVR amino acid sequence pairs selected from the group consisting of SEQ ID NOs: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, 262/264, 266/274, 282/284, 286/288, 290/298, 306/308, 310/312, 314/322, 330/332, 334/336, 338/346, 354/356, 358/360, 362/370, 378/380, 382/384, 386/394, 402/404, 406/408, 410/418, 426/428, 430/432, 434/442, 450/452, 454/456, 458/466, 474/476, 478/480, 482/490, 498/500, 502/504, 506/514, 522/524, 526/528, 530/538, 546/548, 550/552, 554/562, 570/572, 574/576, 578/586, 594/596, 598/600, 602/610, 618/620, 622/624, 626/634, 642/644, 646/648, 650/658, 666/668, 670/672, 674/682, 690/692, 694/696, 698/706, 714/716, 718/720, 722/730, 738/740 and 742/744. In certain exemplary embodiments, the antibody or antigen-binding fragment thereof comprises HCVR/LCVR amino acid sequence pairs of SEQ ID NO: 90/92 or 218/226.

In certain embodiments, the antibody or antigen-binding fragment thereof exhibits one or more or the following properties when administered by weekly subcutaneous injection to an APOE*3Leiden.CETP mouse:
a. reduces total cholesterol levels relative to untreated controls by about 37% when administered at 3 mg/kg;
b. reduces total cholesterol levels relative to untreated controls by about 46% when administered at 10 mg/kg;
c. reduces total cholesterol levels relative to untreated controls by about 48% when administered at 3 mg/kg in combination with 3.6 mg/kg/day atorvastin;
d. reduces total cholesterol levels relative to untreated controls by about 58% when administered at 10 mg/kg in combination with 3.6 mg/kg/day atorvastin;
e. reduces total cholesterol levels by about 36% when administered at 3 mg/kg in combination with 3.6 mg/kg/day atorvastin, relative to treatment with 3.6 mg/kg/day atorvastin alone;
f. reduces total cholesterol levels by about 48% when administered at 10 mg/kg in combination with 3.6 mg/kg/day atorvastin, relative to treatment with 3.6 mg/kg/day atorvastin alone;
g. reduces triglyceride levels relative to untreated controls by about 33% when administered at 3 mg/kg;
h. reduces triglyceride levels relative to untreated controls by about 36% when administered at 10 mg/kg;
i. reduces triglyceride levels by about 40% when administered at 3 mg/kg in combination with 3.6 mg/kg/day atorvastin, relative to treatment with 3.6 mg/kg/day atorvastin;
j. reduces triglyceride levels by about 51% when administered at 10 mg/kg in combination with 3.6 mg/kg/day atorvastin, relative to treatment with 3.6 mg/kg/day atorvastin alone;
k. increases hepatic LDLR expression relative to untreated controls by about 88% when administered at 3 mg/kg;
l. increases hepatic LDLR expression relative to untreated controls by about 178% when administered at 10 mg/kg;
m. increases hepatic LDLR expression by about 71 % when administered at 3 mg/kg in combination with 3.6 mg/kg/day atorvastin, relative to treatment with 3.6 mg/kg/day atorvastin alone;
n. increases hepatic LDLR expression by about 140% when administered at 10 mg/kg in combination with 3.6 mg/kg/day atorvastin, relative to treatment with 3.6 mg/kg/day atorvastin alone;
o. decreases atherosclerotic lesion size relative to untreated controls by about 70% when administered at 3 mg/kg;
p. decreases atherosclerotic lesion size relative to untreated controls by about 87% when administered at 10 mg/kg;
q. decreases atherosclerotic lesion size relative to untreated controls by about 88% when administered at 3 mg/kg in combination with 3.6 mg/kg/day atorvastin;
r. decreases atherosclerotic lesion size relative to untreated controls by about 98% when administered at 10 mg/kg in combination with 3.6 mg/kg/day atorvastin;
s. decreases atherosclerotic lesion size relative by about 82% when administered at 3 mg/kg in combination with 3.6 mg/kg/day atorvastin, relative to treatment with 3.6 mg/kg/day atorvastin alone;
t. decreases atherosclerotic lesion size relative by about 97% when administered at 10 mg/kg in combination with 3.6 mg/kg/day atorvastin, relative to treatment with 3.6 mg/kg/day atorvastin alone;
u. reduces triglyceride levels by about 72% when administered at 3 mg/kg, relative to treatment with 3.6 mg/kg/day atorvastatin alone;
v. reduces triglyceride levels by about 79% when administered at 10 mg/kg, relative to treatment with 3.6 mg/kg/day atorvastatin alone;
w. reduces total cholesterol levels by about 22% when administered at 3 mg/kg, relative to treatment with 3.6 mg/kg/day atorvastatin alone;
x. reduces total cholesterol levels by about 34% when administered at 10 mg/kg, relative to treatment with 3.6 mg/kg/day atorvastatin alone;
y. increases the percentage of undiseased aortic segments by about 236% when administered at 3 mg/kg, relative to untreated control;
z. increases the percentage of undiseased aortic segments by about 549% when administered at 10 mg/kg, relative to untreated control;
aa. increases the percentage of undiseased aortic segments by about 607% when administered at 3 mg/kg in combination with 3.6 mg/kg/day atorvastatin, relative to control; and
bb. increases the percentage of undiseased aortic segments by about 1118% when administered at 3 mg/kg in combination with 3.6 mg/kg/day atorvastatin, relative to control.

### IV. Pharmaceutical Compositions and Methods of Administration

The present invention includes methods which comprise administering a PCSK9 inhibitor to a subject, wherein the PCSK9 inhibitor is contained within a pharmaceutical composition. The pharmaceutical compositions of the invention are formulated with suitable carriers, excipients, and other agents that provide suitable transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

Various delivery systems are known and can be used to administer the pharmaceutical composition of the invention, e.g., encapsulation in liposomes, microparticles, microcapsules, recombinant cells capable of expressing the mutant viruses, receptor mediated endocytosis (see, e.g., Wu et al., 1987, J. Biol. Chem. 262:4429-4432). Methods of administration include, but are not limited to, intradermal, intramuscular, intraperitoneal, intravenous, subcutaneous, intranasal, epidural, and oral routes. The composition may be administered by any convenient route, for example by infusion or bolus injection, by absorption through epithelial or mucocutaneous linings (e.g., oral mucosa, rectal and intestinal mucosa, etc.) and may be administered together with other biologically active agents.

A pharmaceutical composition of the present invention can be delivered subcutaneously or intravenously with a standard needle and syringe. In addition, with respect to subcutaneous delivery, a pen delivery device readily has applications in delivering a pharmaceutical composition of the present invention. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded.

Numerous reusable pen and autoinjector delivery devices have applications in the subcutaneous delivery of a pharmaceutical composition of the present invention. Examples include, but are not limited to AUTOPEN™ (Owen Mumford, Inc., Woodstock, UK), DISETRONICTM pen (Disetronic Medical Systems, Bergdorf, Switzerland), HUMALOG MIX 75/25¹m pen, HUMALOGTM pen, HUMALIN 70/30TM pen (Eli Lilly and Co., Indianapolis, IN), NOVOPENTM I, II and III (Novo Nordisk, Copenhagen, Denmark), NOVOPEN JUNIORTM (Novo Nordisk, Copenhagen, Denmark), BDTM pen (Becton Dickinson, Franklin Lakes, NJ), OPTIPENTM, OPTIPEN PROTM, OPTIPEN STARLETTM, and OPTICLIKTM (sanofi-aventis, Frankfurt, Germany), to name only a few. Examples of disposable pen delivery devices having applications in subcutaneous delivery of a pharmaceutical composition of the present invention include, but are not limited to the SOLOSTARTM pen (sanofi-aventis), the FLEXPEN™ (Novo Nordisk), and the KWIKPEN™ (Eli Lilly), the SURECLICK™ Autoinjector (Amgen, Thousand Oaks, CA), the PENLET™ (Haselmeier, Stuttgart, Germany), the EPIPEN (Dey, L.P.), and the HUMIRA™ Pen (Abbott Labs, Abbott Park IL), to name only a few.

In certain embodiments, the pharmaceutical composition is delivered in a controlled release system. In certain embodiments, a pump may be used (see Langer, supra; Sefton, 1987, CRC Crit. Ref. Biomed. Eng. 14:201). In another embodiment, polymeric materials can be used; see, Medical Applications of Controlled Release, Langer and Wise (eds.), 1974, CRC Pres., Boca Raton, Florida. In yet another embodiment, a controlled release system can be placed in proximity of the composition's target, thus requiring only a fraction of the systemic dose (see, e.g., Goodson, 1984, in Medical Applications of Controlled Release, supra, vol. 2, pp. 115-138). Other controlled release systems are discussed in the review by Langer, 1990, Science 249:1527-1533.

The injectable preparations may include dosage forms for intravenous, subcutaneous, intracutaneous and intramuscular injections, drip infusions, etc. These injectable preparations may be prepared by known methods. For example, the injectable preparations may be prepared, *e.g.,* by dissolving, suspending or emulsifying the antibody or its salt described above *in* a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil], etc. As the oily medium, there are employed, e.g., sesame oil, soybean oil, etc., which may be used in combination with a solubilizing agent such as benzyl benzoate, benzyl alcohol, etc. The, in certain embodiments, injection thus prepared is filled in an appropriate ampoule.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc.

### V. Dosage

The amount of PCSK9 inhibitor *(e.g.,* anti-PCSK9 antibody) administered to a subject according to the methods and compositions of the present invention is, generally, a therapeutically effective amount. As used herein, the phrase "therapeutically effective amount" means a dose of PCSK9 inhibitor that results in a detectable reduction in atherosclerotic lesions. For example, "therapeutically effective amount" of a PCSK9 inhibitor includes, *e.g.,* an amount of PCSK9 inhibitor that causes a reduction of at least 2%, 5%, 10%, 15%, 20%, 25%, 30%, 40%, 50%, 60%, 70%, 80%, 90% or more in sclerotic lesion area or lesion severity when administered to a human patient, *e.g.,* as illustrated in the Examples herein. Alternatively, animal models can be used to establish whether a particular amount of a candidate PCSK9 inhibitor is a therapeutically effective amount.

In the case of an anti-PCSK9 antibody, a therapeutically effective amount can be from about 0.05 mg to about 600 mg, *e.g.,* about 0.05 mg, about 0.1 mg, about 1.0 mg, about 1.5 mg, about 2.0 mg, about 3mg, about 5 mg, about 10 mg, about 15 mg, about 20 mg, about 30 mg, about 40 mg, about 50 mg, about 60 mg, about 70 mg, about 75 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, or about 600 mg, of the anti-PCSK9 antibody.

The amount of anti-PCSK9 antibody contained within the individual doses may be expressed in terms of milligrams of antibody per kilogram of patient body weight (i. e., mg/kg). For example, the anti-PCSK9 antibody may be administered to a patient at a dose of about 0.0001 to about 10 mg/kg of patient body weight.

### VI. Combination Therapies

The methods of the present invention, according to certain embodiments, comprise administering a pharmaceutical composition comprising an anti-PCSK9 antibody to a subject who is on a therapeutic regimen for the treatment of atherosclerosis at the time of, or just prior to, administration of the pharmaceutical composition of the invention. For example, a patient who has previously been diagnosed with atherosclerosis may have been prescribed and is taking a stable therapeutic regimen of another drug prior to and/or concurrent with administration of a pharmaceutical composition comprising an anti-PCSK9 antibody. The prior or concurrent therapeutic regimen may comprise, *e.g.,* (1) an agent which induces a cellular depletion of cholesterol synthesis by inhibiting 3-hydroxy-3-methylglutaryl (HMG)-coenzyme A (CoA) reductase, such as a statin (e.g., cerivastatin, atorvastatin, simvastatin, pitavastatin, rosuvastatin, fluvastatin, lovastatin, pravastatin, etc.); (2) an agent which inhibits cholesterol uptake and or bile acid re-absorption; (3) an agent which increase lipoprotein catabolism (such as niacin); and/or (4) activators of the LXR transcription factor that plays a role in cholesterol elimination such as 22-hydroxycholesterol. In certain embodiments, the patient, prior to or concurrent with administration of an anti-PCSK9 antibody is on a fixed combination of therapeutic agents such as ezetimibe plus simvastatin; a statin with a bile resin (e.g., cholestyramine, colestipol, colesevelam); niacin plus a statin (e.g., niacin with lovastatin); or with other lipid lowering agents such as omega-3-fatty acid ethyl esters (for example, omacor).

### VII. Administration Regimens

According to certain embodiments of the present invention, multiple doses of a PCSK9 inhibitor may be administered to a subject over a defined time course. The methods according to this aspect of the invention comprise sequentially administering to a subject multiple doses of a PCSK9 inhibitor. As used herein, "sequentially administering" means that each dose of PCSK9 inhibitor is administered to the subject at a different point in time, e.g., on different days separated by a predetermined interval (e.g., hours, days, weeks or months). The present invention includes methods which comprise sequentially administering to the patient a single initial dose of a PCSK9 inhibitor, followed by one or more secondary doses of the PCSK9 inhibitor, and optionally followed by one or more tertiary doses of the PCSK9 inhibitor.

The terms "initial dose," "secondary doses," and "tertiary doses," refer to the temporal sequence of administration of the PCSK9 inhibitor. Thus, the "initial dose" is the dose which is administered at the beginning of the treatment regimen (also referred to as the "baseline dose"); the "secondary doses" are the doses which are administered after the initial dose; and the "tertiary doses" are the doses which are administered after the secondary doses. The initial, secondary, and tertiary doses may all contain the same amount of PCSK9 inhibitor, but will generally differ from one another in terms of frequency of administration. In certain embodiments, however, the amount of PCSK9 inhibitor contained in the initial, secondary and/or tertiary doses will vary from one another (e.g., adjusted up or down as appropriate) during the course of treatment.

In certain embodiments, each secondary and/or tertiary dose is administered 1 to 30 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more) days after the immediately preceding dose. The phrase "the immediately preceding dose," as used herein, means, in a sequence of multiple administrations, the dose of PCSK9 inhibitor which is administered to a patient prior to the administration of the very next dose in the sequence with no intervening doses.

In certain embodiments, the methods comprise administering to a patient any number of secondary and/or tertiary doses of a PCSK9 inhibitor. For example, in certain embodiments, only a single secondary dose is administered to the patient. In other embodiments, two or more (e.g., 2, 3, 4, 5, 6, 7, 8, or more) secondary doses are administered to the patient. Likewise, in certain embodiments, only a single tertiary dose is administered to the patient. In other embodiments, two or more (e.g., 2, 3, 4, 5, 6, 7, 8, or more) tertiary doses are administered to the patient.

In embodiments involving multiple secondary doses, each secondary dose may be administered at the same frequency as the other secondary doses. For example, each secondary dose may be administered to the patient 1 to 29 days after the immediately preceding dose. Similarly, in embodiments involving multiple tertiary doses, each tertiary dose may be administered at the same frequency as the other tertiary doses. For example, each tertiary dose may be administered to the patient 1 to 60 days after the immediately preceding dose. Alternatively, the frequency at which the secondary and/or tertiary doses are administered to a patient can vary over the course of the treatment regimen. The frequency of administration may also be adjusted during the course of treatment by a physician depending on the needs of the individual patient following clinical examination.

### EXAMPLES

The following examples are put forth so as to provide those of ordinary skill in the art with a complete disclosure and description of how to make and use the methods and compositions of the invention, and are not intended to limit the scope of what the inventors regard as their invention.

### Example 1: Attainment of Low-Density Lipoprotein Cholesterol Goals in Patients at High Cardiovascular Risk: Results from a Managed Care Population Study

Background: US guidelines support LDL-C goals based on patient's cardiovascular (CV) risk profile, however, little is known regarding real-world patterns of LDL-C goal attainment within specific high CV risk conditions.

Methods: Patients from the OptumInsight IMPACT Database (a large US multi-payer claims database) with an LDL-C measurement during July 2011 to June 2012 and high CV risk conditions were identified. Most recent LDL-C measurement was defined as the index date and high CV risk conditions were identified hierarchically during the pre-index period as follows: recent acute coronary syndrome (ACS, within 6 months pre-index date), coronary events (myocardial infarction, hospitalization for unstable angina, coronary revascularization), stroke, and peripheral vascular disease (PVD).

Results: In total, 110,739 patients met the inclusion criterion. Median (IQR) age was 59 (53 to 65) years, 53.7% were male, and median (IQR) LDL-C was 116 (92 to 143) mg/dL. As of index date, 2.7% had a recent ACS, while 42.1%, 9.2%, and 46.0% had evidence of coronary events, stroke, and PVD, respectively. The following table (Table 1) represents a summary distribution of patients by LDL-C levels as of index date for different high CV risk conditions.

**Table 1: Breakdown of patients by LDL-C level (mg/dL) and risk condition.**

| High CV Risk Condition (% Patients; N) | % Patients by LDL-C Level (mg/dL) | | | | | |
|---|---|---|---|---|---|---|
| | <70 | ≥70 to <100 | ≥100 to <130 | ≥130 to <160 | ≥160 | Any |
| Recent ACS (2.7%; 2,966) | 17.2% | 27.4% | 26.7% | 16.5% | 12.1% | 100.0% |
| Coronary Events (42.1%; 46,616) | 9.5% | 25.1% | 30.3% | 21.4% | 13.8% | 100.0% |
| Stroke (9.2%; 10,240) | 8.0% | 23.8% | 30.4% | 23.5% | 14.3% | 100.0% |
| PVD (46.0%; 50,917) | 6.9% | 23.0% | 31.8% | 23.8% | 14.5% | 100.0% |
| Overall (100%; 110,739) | 8.3% | 24.0% | 30.9% | 22.6% | 14.1% | 100.0% |

Conclusions: In a large, contemporary cohort of high CV risk patients, few achieved LDL-C goals of <70 mg/dL (optional goal for very high CV risk) or <100 mg/dL. Although LDL-C goal achievement improved marginally in patients with conditions signifying higher CV risk, with highest achievement in patients with recent ACS, the majority of these patients were still above LDL-C goals. These data suggest the need for ongoing efforts to address gaps in LDL-C goal attainment and improving CV outcomes in high-risk patients.

### Example 2: Low-Density Lipoprotein Cholesterol Goal Attainment and Lipid-Lowering Therapy in a High Cardiovascular Risk Managed Care Population

Background: While US guidelines support statins as first line therapy to reduce LDL-C, little is known regarding real-world patterns of LDL-C goal attainment with statins and other lipid-lowering therapies (LLTs).

Methods: Patients from the OptumInsight IMPACT Database (a large US multi-payer claims database) with an LDL-C measurement during July 2011 to June 2012 and high risk CV conditions (coronary events (myocardial infarction, hospitalization for unstable angina, coronary revascularization), stroke, and peripheral vascular disease) were identified. LLT prescription was assessed as of most recent LDL-C measurement (index date) and categorized as high-potency statin (atorvastatin 40/80 mg, rosuvastatin 20/40 mg, simvastatin 80 mg), standard-potency statin (other statins), non-statin LLT (ezetimibe, niacin, fibrates, bile acid sequestrants), and no LLT.

Results: In total, 110,739 patients met the inclusion criterion. Median (IQR) age was 59 (53 to 65) years, 53.7% were male, and median (IQR) LDL-C was 116 (92 to 143) mg/dL. As of index date, 10.8% were on high-potency statin, 26.9% were on standard-potency statin, 5.3% were on non-statin LLT, and 57.0% were not on any LLT. The following table (Table 2) represents a summary distribution of patients by LDL-C levels as of index date for different LLT types.

**Table 2: Breakdown of patients by LDL-C level (mg/dL) and LLT type.**

| LLT Type (% Patients; N) | % Patients by LDL-C Level (mg/dL) | | | | | |
|---|---|---|---|---|---|---|
| | <70 | ≥70 to <100 | ≥100 to <130 | ≥130 to <160 | ≥160 | Any |
| High-Potency Statin (10.8%; 12,014) | 15.2% | 33.7% | 26.0% | 13.7% | 11.4% | 100.0% |
| Standard-Potency Statin (26.9%; 29,734) | 11.2% | 30.1% | 28.5% | 18.2% | 12.0% | 100.0% |
| Non-Statin LLT (5.3%; 5,921) | 12.1% | 27.6% | 29.8% | 19.0% | 11.5% | 100.0% |
| No LLT (57.0%; 63,070) | 5.3% | 19.0% | 33.1% | 26.7% | 15.9% | 100.0% |
| Overall (100%; 110,739) | 8.3% | 24.0% | 30.9% | 22.6% | 14.1% | 100.0% |

Conclusions: In this large, contemporary population of patients at high CV risk, few achieved LDL-C goals of <70 mg/dL (optional goal for very high CV risk) or <100 mg/dL. Further, many other patients were not at LDL-C goal despite receiving a high-potency statin. These data suggest the need for ongoing efforts to improve adherence to LLT as well as new therapies to improve goal attainment and CV outcomes.

### Example 3: PCSK-9 monoclonal antibody alirocumab dose-dependently decreases atherosclerosis development and enhances the effects of atorvastatin in APOE*3Leiden.CETP mice.

Background: Serum proprotein convertase subtilisin/kexin type 9 (PCSK9) is a serine protease responsible for low density lipoprotein receptor (LDLR) degradation in the liver, thereby increasing LDL cholesterol levels. PCSK9 inhibition is a potential novel strategy for treatment of CVD, specifically in combination with statins which increase PCSK9 expression. Alirocumab is a fully human PCSK9 monoclonal antibody currently in phase 3 clinical development. Alirocumab comprises the HCVR/LCVR amino acid sequences of SEQ ID NOs: 90/92 and the heavy and light chain CDR amino acid sequences of SEQ ID NOs: 76, 78, 80, 84, 86 and 88 (also referred to as "316P" in US Patent No. 8,062,640).

Methods: Female APOE*3Leiden.CETP mice (described in Westerterp M, et al., Arterioscler Thromb Vase Biol. 2006; 26: 2552-2559., which is incorporated herein in its entirety) at 8-14 weeks for age were used in this study. Mice were fed a Western-type diet (WTD) consisting of 0.15% cholesterol and 15% saturated fat (giving plasma cholesterol levels of approximately 13-17 mM) during a three-week run up period and for the duration of the study.

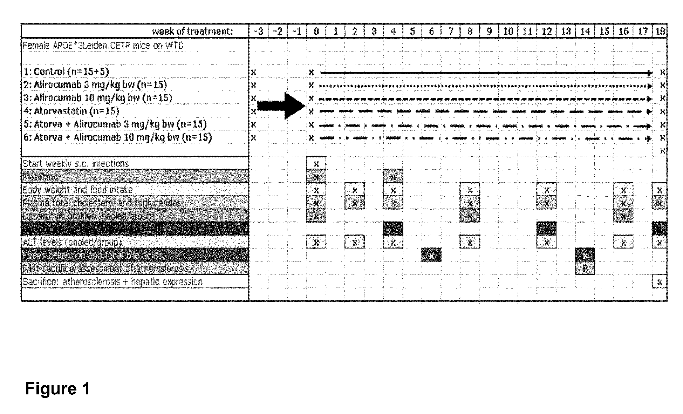

At T=0 weeks, the mice were matched based on body weight, plasma total cholesterol (TC) and triglycerides (TG) after a four-hour fasting period. The mice were then divided into five groups of 15 animals each as set forth in Table 3. The control group contained five extra pilot mice, which were used to assess the development of atherosclerosis at t = 14 weeks. The overall design of the study is graphically represented in Figure 1.

**Table 3: Description of animal treatment groups.**

| Group number | Drug | Administration method | Dosage | Number of animals (n) |
|---|---|---|---|---|
| 1 | Control group (vehicle) | Subcutaneous injection | n/a | 15 + 5 |
| 2 | Alirocumab | subcutaneous injection | 3 mg/kg/wk | 15 |
| 3 | Alirocumab | subcutaneous injection | 10 mg/kg/wk | 15 |
| 4 | Atorvastatin | Admix with food | 3.6 mg/kg/d | 15 |
| 5 | Atorvastatin + Alirocumab | Admix with food + subcutaneous injection | 3.6 mg/kg/d + 3 mg/kg/wk | 15 |
| 6 | Atorvastatin + Alirocumab | Admix with food + subcutaneous injection | 3.6 mg/kg/d + 10 mg/kg/wk | 15 |

Alirocumab was administered through a weekly subcutaneous injection at a dose of 3 or 10 mg/kg with or without atorvastatin (3.6 mg/kg/d administered by diet admix) for 18 weeks. Mice in the control group were injected on a weekly basis with vehicle. At t=14 weeks, the pilot group of control animals (n = 5) was examined for lesion area and severity.

Effects on plasma lipids, hepatic LDLR expression and atherosclerotic lesion size and severity were assessed at various time points up to and including 18 weeks. Data concerning food intake (grams/mouse/day) and body weight (grams) were also collected along with Plasma AST and ALT levels (U/L) (data not shown).

### Results

### i) Effects on plasma lipids

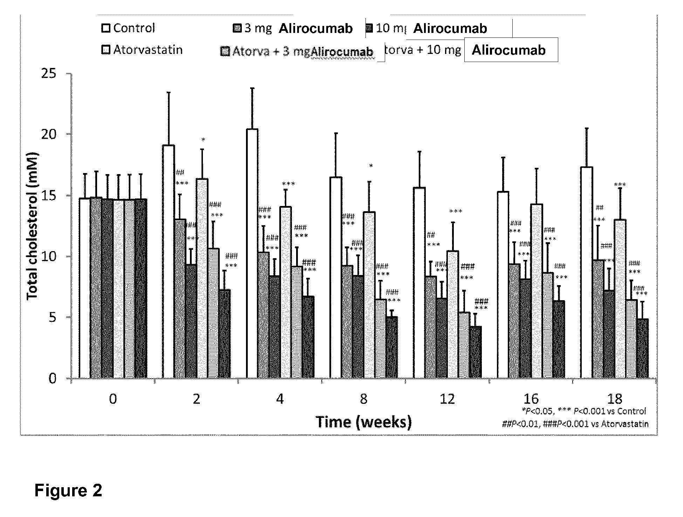

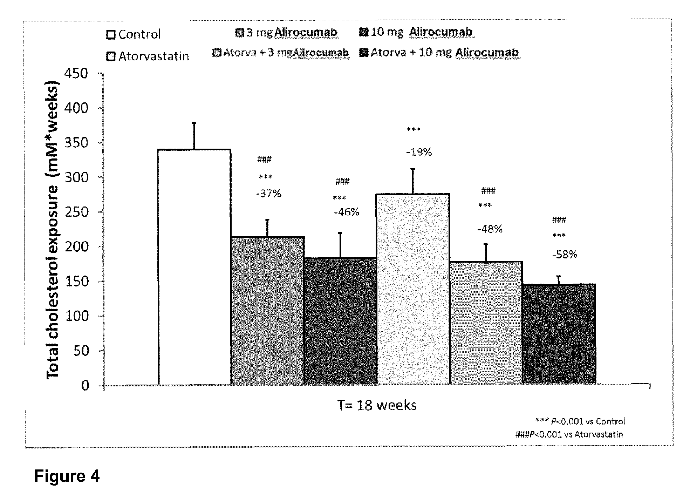

Compared to administration of a control vehicle, Alirocumab dose-dependently decreased total cholesterol (TC) (-37% to -46%, P<0.001) by reduction of the apoB-containing lipoproteins; a further decrease in TC was seen when Alirocumab was administered in combination with atorvastatin (-48% to -58%, P<0.001) (-19% for atorvastatin monotherapy) (Figures 2 and 4). When compared to atorvastatin monotherapy, Alirocumab was also found to decrease TC both as a monotreatment and as a combination therapy with atorvastatin.

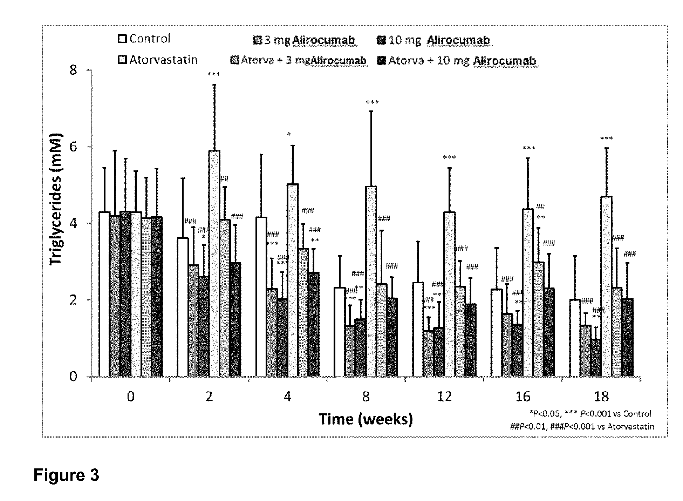

Alirocumab monotreatment dose-dependently decreased triglycerides (TG) as compared to control (-34% to -51 %, P≤0.001 for 10 mg dose) and as compared to atorvastatin monotreatment (-72% to -79%, P<0.001) by reduction of the apoB-containing lipoproteins. The combination treatment reduced TG (-51% and -57%, P<0.001) to a greater extent as compared to atorvastatin mono-treatment (Figure 3).

### ii) Effects on Hepatic LDLR expression as measured by Western blot analysis

Liver pieces were homogenized in lysis buffer (50 mM Tris-HCL (pH=7.4), 150 mM NaCl, 0.25% deoxycholic acid, 1% NP-40 (Igepal), 1mM EDTA, protease inhibitor cocktail (complete, Roche), 1 mM PMSF, 1 mM Na3VO4). Protein concentration in cell lysates was determined by bicinchonic acid (BCA) protein assay (Thermo scientific) according to manufacturer's instructions. 50 µg of protein lysates were separated by SDS -PAGE under reducing conditions, and blotted to polyvinylidene difluoride (PVDF). Blots were hybridized with Goat-anti-mouse-LDLR from R&D systems and Rabbit-anti-goat-HRP from AbD Serotec or Mouse-anti-α-Tubulin from Sigma and Horse-anti-mouse-HRP from Cell Signaling Technologies according to manufacturer's instructions and subsequently developed with West Femto Super Signal ECL (Thermo scientific) and imaged with the Chemi Doc-it system. Intensities of protein bands were quantified using Image J software.

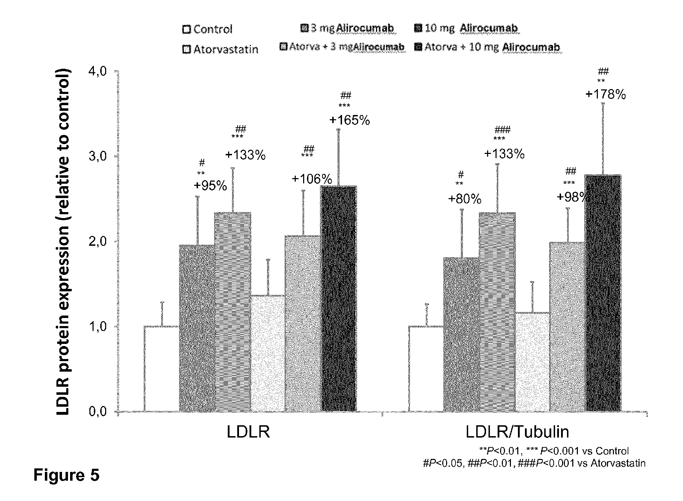

Rescue of LDLR degradation was verified by an increase in hepatic LDLR expression after Alirocumab treatment (+95% to +133%, P≤0.005). The combination treatment also increased hepatic LDLR expression (+106% and +165%, P≤0.003) to a greater extent as compared to atorvastatin mono-treatment (Figure 5).

### iii) Effects on atherosclerotic lesion size and severity

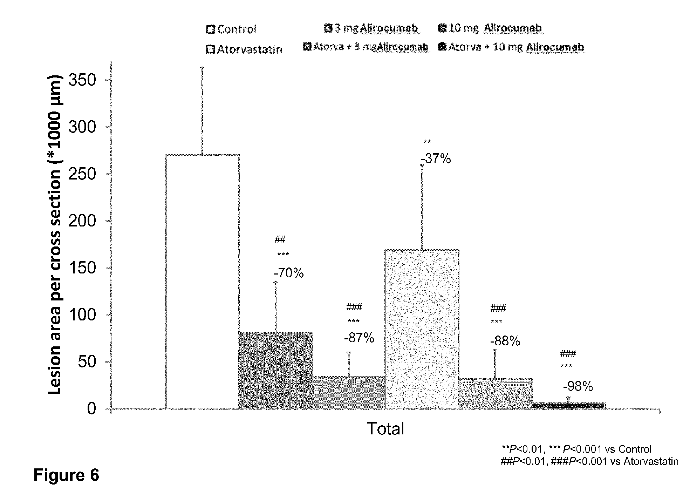

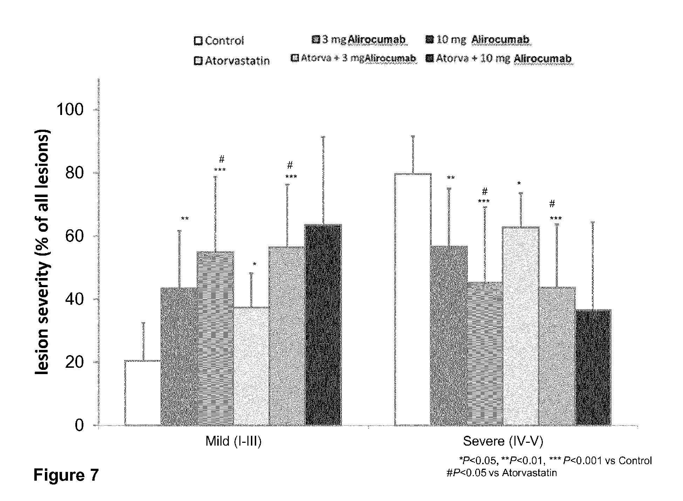

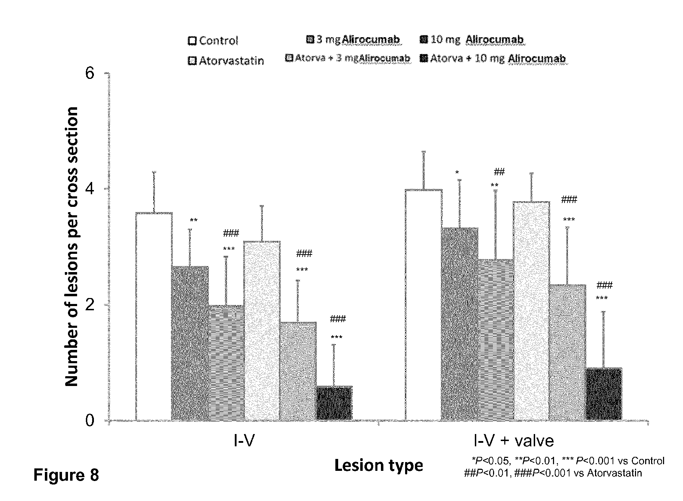

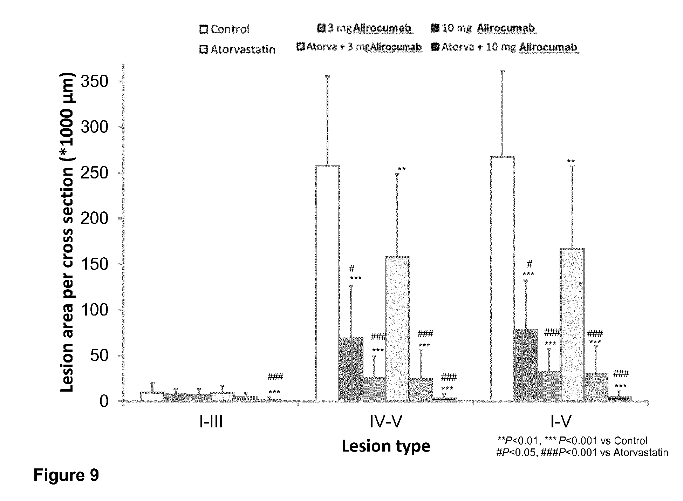

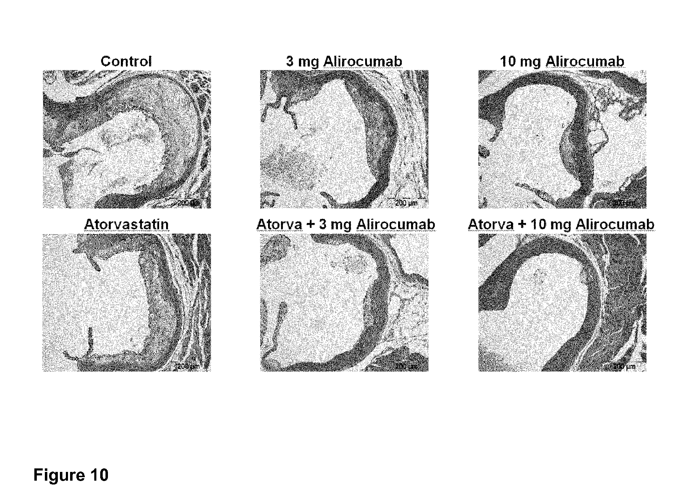

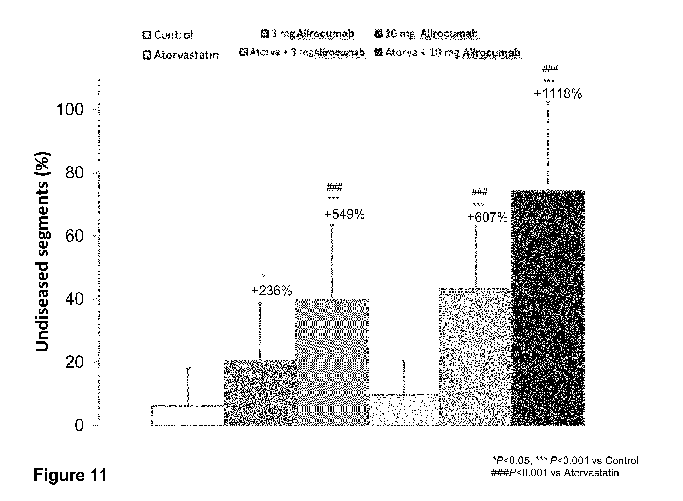

Atherosclerotic lesion size and severity were determined according to van der Hoorn et al. (2009), British Journal of Pharmacology, 156, 1067-1075, which is incorporated herein by reference in its entirety. Briefly, mice were sacrificed and their hearts were dissected, formalin-fixed, and embedded in paraffin. Serial cross sections were taken through the aortic valve area (5µm) for analysis. Four sections at intervals of 50µm were stained with HPS (haematoxylin-phloxin-saffrom) and used for quantitative and qualitative assessment of the atherosclerotic lesions. Lesions were classified according to Verschuren et al. (2005) into the following categories: (i) early fatty streak; (ii) regular fatty streak; (iii) mild lesion; (iv) moderate lesion; or (v) severe lesion. The percentages of all lesions found in the respective categories were calculated for each mouse. The total lesion area was calculated per cross section. Statistical differences were assessed by using the non-parametrical Kruskal-Wallis test followed by Mann-Whitney U-test. Alirocumab alone dose-dependently decreased atherosclerotic lesion size (-70% and -87%, P<0.001) and severity and administered in combination with atorvastatin enhanced the effects on lesion size (-88% and -98%, P<0.001) as compared to control (Figures 6-10). When compared to atorvastatin mono-treatment, the combination treatment further decreased lesion size (-82% and -97%, P≤0.002) and severity. There was also a significant increase in the percentage of undiseased segments seen in animals treated with Alirocumab alone (+236% and +549%) or in combination with atorvastatin (+607% and +1118%) as compared to control that was not seen in animals treated with atorvastatin alone (Figure 11).

## Claims

1. A pharmaceutical composition comprising a PCSK9 inhibitor for use in the inhibition of atherosclerotic plaque formation in a subject.

2. A method of inhibiting atherosclerotic plaque formation in a subject, the method comprising administering to the subject a pharmaceutical composition comprising a PCSK9 inhibitor.

3. The pharmaceutical composition or method of claim 1 or 2, wherein the subject is nonhyperlipidemic.

4. The pharmaceutical composition or method of claim 1 or 2, wherein the subject is apparently healthy.

5. A method of treating or inhibiting progression of atherosclerosis in a subject, the method comprising:
a. selecting a subject who has suffered a stroke or myocardial infarction; and
b. administering to the subject a pharmaceutical composition comprising a PCSK9 inhibitor, thereby treating or inhibiting progression of atherosclerosis.

6. The method of claim 5, wherein the subject is nonhyperlipidemic.

7. A method of treating or inhibiting progression of atherosclerosis in a nonhyperlipidemic subject, the method comprising:
a. selecting a subject who has, or is known to be at risk of developing, atherosclerosis, wherein the subject is nonhyperlipidemic; and
b. administering to the subject a pharmaceutical composition comprising a PCSK9 inhibitor, thereby treating or inhibiting progression of atherosclerosis.

8. The method of claim 7, wherein the subject is apparently healthy.

9. The pharmaceutical composition or method of any one of the preceding claims, wherein the subject is nonhypercholesterolemic.

10. The pharmaceutical composition or method of any one of the preceding claims, wherein the subject is nonhypertriglyceridemic.

11. The pharmaceutical composition or method of any one of the preceding claims, wherein the subject has a disease or disorder selected from the group consisting of type I diabetes mellitus, type II diabetes mellitus, Kawasaki disease, chronic inflammatory disease, and hypertension.

12. The pharmaceutical composition or method of any one of the preceding claims, wherein the subject has elevated levels of an inflammatory marker.

13. The pharmaceutical composition or method of claim 12, wherein the inflammatory marker is C-reactive protein.

14. The pharmaceutical composition or method of claim 12, wherein the inflammatory marker is an inflammatory cytokine.

15. The pharmaceutical composition or method of any one of the preceding claims, wherein the PCSK9 inhibitor is an antibody, or antigen binding fragment thereof, that specifically binds to PCSK9.

16. The pharmaceutical composition or method of claim 15, wherein the antibody, or antigen binding fragment thereof, exhibits one or more or the following properties when administered by weekly subcutaneous injection to a APOE*3Leiden.CETP mouse:
a. reduces total cholesterol levels relative to untreated controls by about 37% when administered at 3 mg/kg;
b. reduces total cholesterol levels relative to untreated controls by about 46% when administered at 10 mg/kg;
c. reduces total cholesterol levels relative to untreated controls by about 48% when administered at 3 mg/kg in combination with 3.6 mg/kg/day atorvastin;
d. reduces total cholesterol levels relative to untreated controls by about 58% when administered at 10 mg/kg in combination with 3.6 mg/kg/day atorvastin;
e. reduces total cholesterol levels by about 36% when administered at 3 mg/kg in combination with 3.6 mg/kg/day atorvastin, relative to treatment with 3.6 mg/kg/day atorvastin alone;
f. reduces total cholesterol levels by about 48% when administered at 10 mg/kg in combination with 3.6 mg/kg/day atorvastin, relative to treatment with 3.6 mg/kg/day atorvastin alone;
g. reduces triglyceride levels relative to untreated controls by about 34% when administered at 3 mg/kg;
h. reduces triglyceride levels relative to untreated controls by about 51% when administered at 10 mg/kg;
i. reduces triglyceride levels by about 51% when administered at 3 mg/kg in combination with 3.6 mg/kg/day atorvastin, relative to treatment with 3.6 mg/kg/day atorvastin;
j. reduces triglyceride levels by about 57% when administered at 10 mg/kg in combination with 3.6 mg/kg/day atorvastin, relative to treatment with 3.6 mg/kg/day atorvastin alone;
k. increases hepatic LDLR expression relative to untreated controls by about 95% when administered at 3 mg/kg;
l. increases hepatic LDLR expression relative to untreated controls by about 133% when administered at 10 mg/kg;
m. increases hepatic LDLR expression by about 106% when administered at 3 mg/kg in combination with 3.6 mg/kg/day atorvastin, relative to treatment with 3.6 mg/kg/day atorvastin alone;
n. increases hepatic LDLR expression by about 165% when administered at 10 mg/kg in combination with 3.6 mg/kg/day atorvastin, relative to treatment with 3.6 mg/kg/day atorvastin alone;
o. decreases atherosclerotic lesion size relative to untreated controls by about 70% when administered at 3 mg/kg;
p. decreases atherosclerotic lesion size relative to untreated controls by about 87% when administered at 10 mg/kg;
q. decreases atherosclerotic lesion size relative to untreated controls by about 88% when administered at 3 mg/kg in combination with 3.6 mg/kg/day atorvastin;
r. decreases atherosclerotic lesion size relative to untreated controls by about 98% when administered at 10 mg/kg in combination with 3.6 mg/kg/day atorvastin;
s. decreases atherosclerotic lesion size relative by about 82% when administered at 3 mg/kg in combination with 3.6 mg/kg/day atorvastin, relative to treatment with 3.6 mg/kg/day atorvastin alone;
t. decreases atherosclerotic lesion size relative by about 97% when administered at 10 mg/kg in combination with 3.6 mg/kg/day atorvastin, relative to treatment with 3.6 mg/kg/day atorvastin alone;
u. reduces triglyceride levels by about 72% when administered at 3 mg/kg, relative to treatment with 3.6 mg/kg/day atorvastatin alone;
v. reduces triglyceride levels by about 79% when administered at 10 mg/kg, relative to treatment with 3.6 mg/kg/day atorvastatin alone;
w. reduces total cholesterol levels by about 22% when administered at 3 mg/kg, relative to treatment with 3.6 mg/kg/day atorvastatin alone;
x. reduces total cholesterol levels by about 34% when administered at 10 mg/kg, relative to treatment with 3.6 mg/kg/day atorvastatin alone;
y. increases the percentage of undiseased aortic segments by about 236% when administered at 3 mg/kg, relative to untreated control;
z. increases the percentage of undiseased aortic segments by about 549% when administered at 10 mg/kg, relative to untreated control;
aa. increases the percentage of undiseased aortic segments by about 607% when administered at 3 mg/kg in combination with 3.6 mg/kg/day atorvastatin, relative to control; and
bb. increases the percentage of undiseased aortic segments by about 1118% when administered at 3 mg/kg in combination with 3.6 mg/kg/day atorvastatin, relative to control.

17. The pharmaceutical composition or method of claims 15 or 16, wherein the antibody comprises: a heavy chain variable domain comprising the CDR amino acid sequences set forth in SEQ ID NOs: 76, 78, and 80; and a light chain variable domain comprising the CDR amino acid sequences set forth in SEQ ID NOs: 84, 86 and 88.

18. The pharmaceutical composition or method of claims 15 or 16, wherein the antibody comprises: a heavy chain variable domain comprising the CDR amino acid sequences set forth in SEQ ID NOs: 220, 222, and 224; and a light chain variable domain comprising the CDR amino acid sequences set forth in SEQ ID NOs: 802, 230 and 232.

19. The pharmaceutical composition or method of claims 15 or 16, wherein the antibody comprises the heavy chain variable domain and the light chain variable domain respectively comprise the amino acid sequences set forth in SEQ ID NOs 90 and 92.

20. The pharmaceutical composition or method of claims 15or 16, wherein the antibody comprises the heavy chain variable domain and the light chain variable domain respectively comprise the amino acid sequences set forth in SEQ ID NOs 218 and 216.

21. The pharmaceutical composition or method of any one of claims 15-20, wherein the antibody or antigen-binding fragment thereof binds to the same epitope on PCSK9 as an antibody comprising the heavy and light variable domain amino acid sequences forth in SEQ ID NOs: 90 and 92; or 218 and 216, respectively.

22. The pharmaceutical composition or method of any one of claims 15-20, wherein the antibody or antigen-binding fragment thereof competes for binding to PCSK9 with an antibody comprising the heavy and light chain variable domain amino acid sequences forth in SEQ ID NOs: 90 and 92; or 218 and 216, respectively.

23. The pharmaceutical composition or method of any one of the preceding claims, wherein the PCSK9 inhibitor reduces atherosclerotic plaque formation in the subject by at least 10% [optionally 20%, 30%, 40%, 50%, 60%, 70%, 80%, or 90%].

24. The pharmaceutical composition or method of any one of the preceding claims, wherein the PCSK9 inhibitor is administered in combination with a statin.

25. The pharmaceutical composition or method of 24, wherein the therapeutic statin is selected from the group consisting of cerivastatin, atorvastatin, simvastatin, pitavastatin, rosuvastatin, fluvastatin, lovastatin and pravastatin.

26. The pharmaceutical composition or method of 25, wherein the statin is atorvastatin.
